Europäisches Patentamt

European Patent Office

Office européen des brevets

(19)

(11) Veröffentlichungsnummer: **0 382 049 B1**

# (12) EUROPÄISCHE PATENTSCHRIFT

(45) Veröffentlichungstag der Patentschrift:
**18.12.91 Patentblatt 91/51**

(51) Int. Cl.⁵: **B01J 23/74, C07C 209/16, C07D 295/02, C07C 213/02**

(21) Anmeldenummer: **90101784.8**

(22) Anmeldetag: **30.01.90**

(54) Katalysator und Verfahren zur hydrierenden Aminierung von Alkoholen.

(30) Priorität: **04.02.89 DE 3903367**

(43) Veröffentlichungstag der Anmeldung:
**16.08.90 Patentblatt 90/33**

(45) Bekanntmachung des Hinweises auf die
Patenterteilung:
**18.12.91 Patentblatt 91/51**

(84) Benannte Vertragsstaaten:
**BE CH DE ES FR GB IT LI NL SE**

(56) Entgegenhaltungen:
**EP-A- 0 017 651**
**EP-A- 0 254 335**
**GB-A- 2 148 287**
**US-A- 4 772 750**

(73) Patentinhaber: **BASF Aktiengesellschaft**
**Carl-Bosch-Strasse 38**
**W-6700 Ludwigshafen (DE)**

(72) Erfinder: **Irgang, Matthias, Dr.**
**Andreas-Hofer-Weg 41**
**W-6900 Heidelberg (DE)**
Erfinder: **Schossig, Juergen, Dr.**
**Raiffeisenstrasse 16**
**W-6701 Fussgoenheim (DE)**
Erfinder: **Schroeder, Wolfgang, Dr.**
**Seebacher Strasse 51**
**W-6702 Bad Duerkheim (DE)**
Erfinder: **Winderl, Siegfried, Dr.**
**In der Neckarhelle 126**
**W-6900 Heidelberg (DE)**

EP 0 382 049 B1

## Beschreibung

Die vorliegende Erfindung betrifft Katalysatoren, deren aktive Masse neben 20 bis 85 Gew.% sauerstoffhaltigen Zirkoniumverbindungen, berechnet als $ZrO_2$, 1 bis 30 Gew.% sauerstoffhaltige Verbindungen des Kupfers, berechnet als CuO und jeweils 1 bis 40 Gew.% sauerstoffhaltige Verbindungen des Cobalts und Nickels, berechnet als CoO bzw. NiO, enthält.

Des weiteren betrifft die vorliegende Erfindung ein Verfahren zur katalytischen, hydrierenden Aminierung von Alkoholen mit Ammoniak in Gegenwart von Wasserstoff, bei erhöhter Temperatur und bei erhöhtem Druck.

Aus der DE-A-1953263 ist es bekannt, Amine durch die hydrierende Aminierung der entsprechenden Alkohole an Cobalt, Nickel und Kupfer enthaltenden Trägerkatalysatoren herzustellen. Als Trägermaterial wird in diesen Katalysatoren Aluminiumoxid oder Siliciumdioxid verwendet. Mit diesen Katalysatoren lassen sich gute Umsätze erzielen, vorausgesetzt, es werden relativ hohe Temperaturen und Drucke angewandt. Wird bei tieferen Temperaturen und Drucken gearbeitet, so geht bei diesen Katalysatoren der Umsatz stark zurück, und das gleiche gilt für die Selektivität dieser Katalysatoren bei der Herstellung bestimmter Aminierungsprodukte.

Nach der Lehre der EP-A-254335 werden Ni/Co/Ru-Katalysatoren auf Aluminiumoxid- oder Siliciumdioxid-Trägern, welche zusätzlich noch Halogenide in ihrer aktiven Masse enthalten, zur hydrierenden Aminierung von Alkoholen verwendet. Doch auch mit diesen Katalysatoren werden bei 200°C und einem Druck von 55 bar nur Umsätze von maximal 61% erzielt, wobei das Reaktionsprodukt bis zu ca. 90% aus primären Aminen besteht. Dialkylamine können mit diesem Katalysator nur in geringen Mengen erhalten werden.

In der US-A 4151204 sind Katalysatoren zur speziellen Herstellung von Aminoalkoholen beschrieben. Diese Katalysatoren, welche aus einem Metall wie Cobalt, Nickel oder Kupfer, bevorzugt aus Nickel oder Cobalt bestehen, können zusätzlich noch mit unter anderem geringen Mengen an Zirkonium dotiert sein, wobei das Zirkonium bezüglich des Nickels oder Cobalts in einem Atomverhältnis von 0,005 bis 0,2 zugesetzt wird. Höhere Zirkoniumgehalte führen nach der Lehre dieser Patentschrift zu Nebenreaktionen wie der Zersetzung der Produkte.

Da Amine vielfältig genutzte und in großen Mengen benötigte Zwischenprodukte der chemischen Industrie sind, lag der Erfindung die Aufgabe zugrunde, die Wirtschaftlichkeit bisheriger Verfahren zur hydrierenden Aminierung von Alkoholen zu verbessern. Zu diesem Zweck sollten Katalysatoren gefunden werden, die es erlauben, die hydrierende Aminierung von Alkoholen auch bei relativ niedrigen Temperaturen und Drucken mit hohem Umsatz, guter Ausbeute und Selektivität durchzuführen.

Dementsprechend wurden Katalysatoren gefunden, deren aktive Masse neben 20 bis 85 Gew.% sauerstoffhaltigen Zirkoniumverbindungen, berechnet als $ZrO_2$, 1 bis 30 Gew.% sauerstoffhaltige Verbindungen des Kupfers, berechnet als CuO und jeweils 1 bis 40 Gew.% sauerstoffhaltige Verbindungen des Cobalts und Nickels, berechnet als CoO bzw. NiO, enthält.

Des weiteren wurde ein Verfahren zur katalytischen, hydrierenden Aminierung von Alkoholen mit Ammoniak in Gegenwart von Wasserstoff, bei erhöhter Temperatur und bei erhöhtem Druck gefunden, das dadurch gekennzeichnet ist, daß man als Katalysator einen der in den Ansprüchen definierten Katalysatoren verwendet.

Im allgemeinen werden die erfindungsgemäßen Katalysatoren bevorzugt in Form von Vollkatalysatoren eingesetzt. Mit dem Begriff "Vollkatalysator" wird ein Katalysator bezeichnet, der im Gegensatz zu einem Trägerkatalysator nur aus katalytisch aktiver Masse besteht. Vollkatalysatoren können dergestalt eingesetzt werden, daß man die katalytisch aktive, zu Pulver vermahlene Masse in das Reaktionsgefäß einbringt oder, daß man die katalytisch aktive Masse nach Mahlung, Vermischung mit Formhilfsmitteln, Formung und Temperung als Katalysatorformkörper — beispielsweise als Kugeln, Zylinder, Ringe, Spiralen — im Reaktor anordnet.

Die katalytisch aktive Masse der erfindungsgemäßen Katalysatoren enthält neben sauerstoffhaltigen Verbindungen des Zirkoniums, sauerstoffhaltige Verbindungen des Cobalts, Nickels und Kupfers.

Da sich die Konzentrationsangaben jeweils — falls nicht anders angegeben — auf die katalytisch aktive Masse des Katalysators beziehen, wird die katalytisch aktive Masse des Katalysators im folgenden als die Summe der Massen der katalytisch aktiven Bestandteile Zirkonium, Cobalt, Nickel und Kupfer im Katalysator, jeweils berechnet als $ZrO_2$, CoO, NiO bzw. CuO, nach dessen letzter Wärmebehandlung und vor dessen Reduktion mit Wasserstoff, definiert.

Im allgemeinen beträgt der Zirkoniumoxidgehalt der erfindungsgemäßen Katalysatoren zwischen 20 und 85, bevorzugt 70 bis 80 Gew.%.

Die anderen Komponenten Cobalt, Nickel und Kupfer sind im allgemeinen insgesamt in Mengen von 15 bis 80, bevorzugt 15 bis 60, insbesondere 15 bis 50 Gew.% in der katalytisch aktiven Masse enthalten.

Bevorzugte Katalysatoren enthalten in ihrer katalytisch aktiven Masse 20 bis 85 Gew.% sauerstoffhaltige Zirkoniumverbindungen, 1 bis 30 Gew.% sauerstoffhaltige Kupferverbindungen und jeweils 1 bis 40 Gew.% sauerstoffhaltige Verbindungen des Cobalts und Nickels.

Besonders bevorzugt unter den erfindungsgemäß hergestellten Katalysatoren sind solche, welche in der

2

aktiven Masse neben 70 bis 80 Gew.% sauerstoffhaltigen Zirkoniumverbindungen, 1 bis 10 Gew.% sauerstoffhaltige Kupferverbindungen und jeweils 5 bis 20 Gew.% sauerstoffhaltige Cobalt- und Nickelverbindungen enthalten.

Zur Herstellung der Vollkatalysatoren sind verschiedenerlei Verfahrensweisen möglich. Sie sind beispielsweise durch Anteigen pulvriger Mischungen der Hydroxide, Carbonate, Oxide und/oder anderer Salze der Komponenten Zirkonium, Cobalt, Nickel und Kupfer mit Wasser und nachfolgendes Extrudieren und Tempern der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Cobalt-, Nickel- und Kupferkomponenten aus einer diese Elemente enthaltenden, wäßrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate-, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden, vorteilhaft werden diese Aufschlämmungen durch Ausfällen der schwerlöslichen Zirkoniumverbindungen aus wäßrigen Zirkoniumsalzlösungen mittels Mineralbasen erhalten.

Bevorzugt werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wäßrige Salzlösung in der Wärme und unter Rühren so lange mit einer wäßrigen Mineralbase, insbesondere einer Alkalimetallbase — beispielsweise Natriumcarbonat, Natriumhydroxid, Kaliumcarbonat oder Kaliumhydroxid — versetzt, bis die Fällung vollständig ist. Die Art der verwendeten Salze ist im allgemeinen nicht kritisch — da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Erfindungsgemäße Katalysatoren mit besonders vorteilhaften Eigenschaften sind dadurch erhältlich, daß man einen Teil der Zirkoniumkomponente des Katalysators, zweckmäßigerweise aus einer wäßrigen Zirkoniumsalzlösung, separat in einer Fällungsapparatur durch Zugabe wäßriger Mineralbasen fällt. Auf das so erhaltene, vorzugsweise frisch gefällte Zirkoniumoxid-Hydrat, kann dann der restliche Teil der Zirkoniumkomponente des Katalysators zusammen mit den anderen katalytisch aktiven Komponenten in einer Mischfällung gefällt werden, wie oben beschrieben wurde. Dabei erweist es sich in der Regel als besonders zweckmäßig 10 bis 80 Gew.% vorzugsweise 30 bis 70 Gew.% und insbesondere 40 bis 60 Gew.% der Gesamtzirkoniummenge der katalytisch aktiven Masse vorzufällen.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der genannten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet : Nach dem Waschen werden sie im allgemeinen bei 80 bis 200°C, vorzugsweise bei 100 bis 150°C, getrocknet und danach calciniert. Die Calcinierung wird im allgemeinen bei Temperaturen zwischen 300 und 800°C, vorzugsweise bei 400 bis 600°C, insbesondere bei 450 bis 550°C ausgeführt.

Nach der Calcinierung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Tablettenpresse zu Formlingen verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur hydrierenden Aminierung von Alkoholen werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200°C über einen Zeitraum von 12 bis 20 Stunden einer Stickstoff-/Wasserstoff-Atmosphäre ausgesetzt und anschließend noch bis zu ca. 24 Stunden bei 200 bis 300°C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form des Katalysators vorliegen.

Die erfindungsgemäß hergestellten Katalysatoren eignen sich insbesondere als Katalysatoren zur hydrierenden Aminierung aliphatischer Alkohole. Bei dieser Reaktion werden Alkohole in Gegenwart eines Aminierungsmittels - z.B. Ammoniak, Mono- oder Dialkylamine - und in Gegenwart von Wasserstoff mit Hilfe eines wie ein Hydrier-/Dehydrierkatalysator wirkenden Katalysators in die entsprechenden Amine umgewandelt. Zum Reaktionsmechanismus dieser Reaktion wird angenommen, daß der eingesetzte Alkohol zunächst zur entsprechenden Carbonylverbindung dehydriert wird, diese dann mit Ammoniak oder dem Amin zu dem entsprechenden Azomethin oder Enamin kondensiert, welches dann im letzten Schritt der Reaktionsfolge zu dem entsprechenden Amin hydriert wird. Daraus folgt, daß bei der hydrierenden Aminierung anstelle der Alkohole auch die ihnen entsprechenden Carbonylverbindungen mit dem gleichen Erfolg eingesetzt werden können. Der Einsatz von Carbonylverbindungen in die hydrierende Aminierung ist somit der Anwendung der entsprechenden Alkohole äquivalent. Lediglich aufgrund ihrer leichten Verfügbarkeit und Preiswürdigkeit werden Alkohole im allgemeinen bevorzugt zu dieser Reaktion verwendet.

Als Ausgangsstoffe eignen sich praktisch alle primären und sekundären aliphatischen Alkohole. Die aliphatischen Alkohole können geradkettig, verzweigt oder cyclisiert sein. Sekundäre Alkohole werden ebenso aminiert wie primäre Alkohole. Hinsichtlich der Kohlenstoffzahl der aminierbaren Alkohole sind bisher ebenfalls keine Beschränkungen bekannt. Die Alkohole können ferner Substituenten tragen, welche sich unter den Bedingungen der hydrierenden Aminierung inert verhalten, beispielsweise Alkoxi- oder Alkylenoxygruppen. Sollen mehrbasische Alkohole aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Alkohole aminiert : Methanol, Ethanol, Propanol, Isopropanol, Butanol, Pentanol, Hexanol, 2-Ethylhexanol, Tridecanol, Stearylalkohol, Palmitylalkohol, Cyclohexanol, Ethanolamin, Propanolamin, Isopropanolamin, Hexanolamin, Diethanolamin, N-Alkyldiethanolamine, Diisopropanolamin, Ethylenglykol, Propylenglykol, Butandiol, Pentandiol, Hexandiol, 4,4′-Bishydroxycyclohexylpropan-(2,2), Methoxyethanol, Ethoxyethanol, Propoxyethanol, Butoxyethanol, Polyethylenglykolether, Polypropylenglykolether und Polybutylenglykolether. Die letztgenannten Polyalkylenglykolether werden bei der erfindungsgemäßen Umsetzung durch Umwandlung ihrer freien Hydroxylgruppen zu den entsprechenden Aminen umgewandelt.

Als Aminierungsmittel bei der hydrierenden Aminierung von Alkoholen können sowohl Ammoniak als auch primäre oder sekundäre, aliphatische oder cycloaliphatische Amine verwendet werden.

Bei Verwendung von Ammoniak als Aminierungsmittel werden die alkoholischen Hydroxylgruppen zunächst in freie Aminogruppen ($-NH_2$) umgewandelt. Die so gebildeten primären Amine können mit weiterem Alkohol zu den entsprechenden sekundären Aminen und diese wiederum mit weiterem Alkohol zu den entsprechenden, symmetrischen tertiären Aminen reagieren. Je nach Zusammensetzung des Reaktionsansatzes und je nach den angewandten Reaktionsbedingungen—Druck, Temperatur, Reaktionszeit—lassen sich auf diese Weise je nach Wunsch bevorzugt primäre, sekundäre oder tertiäre Amine darstellen.

Aus mehrbasischen Alkoholen lassen sich auf diese Weise durch intramolekulare hydrierende Aminierung cyclische Amine wie Pyrrolidine, Piperidine, Piperazine und Morpholine herstellen.

Ebenso wie Ammoniak lassen sich primäre oder sekundäre Amine als Aminierungsmittel verwenden. Bevorzugt werden diese Aminierungsmittel zur Herstellung unsymmetrisch substituierter Di- oder Trialkylamine, wie Ethyldiisopropylamin und Ethyldicyclohexylamin verwendet. Bevorzugt werden beispielsweise die folgenden Mono- und Dialkylamine als Aminierungsmittel verwendet : Methylamin, Dimethylamin, Ethylamin, Diethylamin, Propylamin, Diisopropylamin, Butylamin, Pentylamin, Hexylamin und Cyclohexylamin.

Das Aminierungsmittel kann bezüglich der zu aminierenden alkoholischen Hydroxylgruppe in stöchiometrischer Menge eingesetzt werden. Bevorzugt wird jedoch mit einem Überschuß an Aminierungsmittel gearbeitet und zwar im allgemeinen mit einem mehr als 5 molaren Überschuß pro Mol zu aminierender alkoholischer Hydroxylgruppe. Speziell Ammoniak wird im allgemeinen mit einem 5 bis 250-fachen, bevorzugt 10 bis 100-fachen, insbesondere 25 bis 80-fachen molaren Überschuß pro Mol umzusetzender alkoholischer Hydroxylgruppen eingesetzt. Höhere Überschüsse sowohl an Ammoniak als auch an primären oder sekundären Aminen sind möglich.

Der Wasserstoff wird der Reaktion im allgemeinen in einer Menge von 5 bis 400 Nl, bevorzugt in einer Menge von 50 bis 200 Nl pro Mol Alkoholkomponente zugeführt.

Die Umsetzung erfolgt im allgemeinen ohne zusätzliches Lösungsmittel. Bei der Umsetzung hochmolekularer, hochviskoser oder bei Raumtemperatur fester Ausgangsverbindungen oder Produkte kann es vorteilhaft sein, ein unter den Reaktionsbedingungen inertes Lösungsmittel, wie Tetrahydrofuran, Dioxan, N-Methylpyr-

EP 0 382 049 B1

rolidon oder Ethylenglykoldimethylether mitzuverwenden.

Üblicherweise arbeitet man bei der Umsetzung bei Temperaturen oberhalb 100°C, besonders oberhalb 170°C und bevorzugt den Temperaturbereich von 180 bis 230°C. Im allgemeinen wird die Reaktion bei einem Druck von mehr als 10 bar ausgeführt, bevorzugt werden jedoch Drucke von 30 bis 400 bar, insbesondere von 30 bis 300 bar angewandt.

Die Anwendung höherer Temperaturen und eines höheren Gesamtdruckes ist möglich. Der Gesamtdruck im Reaktionsgefäß, welches sich aus der Summe der Partialdrücke des Aminierungsmittels, der Alkoholkomponente und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck eingestellt.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesondere 40 bis 50 Volumenteile betragen.

Praktisch geht man bei der Durchführung der Umsetzung im allgemeinen so vor, daß man dem Katalysator, der sich üblicherweise in einem bevorzugt von außen beheizten Festbettreaktor befindet, bei der gewünschten Reaktionstemperatur und dem gewünschten Druck den Alkohol und das Aminierungsmittel simultan zuführt. Dabei belastet man den Katalysator im allgemeinen mit 0,02 bis 0,5, bevorzugt 0,05 bis 0,3 und besonders bevorzugt mit 0,08 bis 0,15 l Alkohol pro Liter Katalysator und Stunde. Hierbei ist es zweckmäßig, die Reaktanten bereits vor der Zuführung in das Reaktionsgefäß zu erwärmen und zwar bevorzugt auf die Reaktionstemperatur.

Der Reaktor kann sowohl in der Sumpf- als auch in der Rieselfahrweise betrieben werden, d.h. man kann die Reaktanten sowohl von unten nach oben als auch von oben nach unten durch den Reaktor leiten. Es versteht sich von selbst, daß sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden, nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht oder nicht vollständig umgesetzte Alkoholkomponente.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

Die auf diese Weise erhältlichen Amine finden u.a. als Zwischenprodukte bei der Herstellung von Tensiden, Arznei- und Pflanzenschutzmitteln sowie von Vulkanisationsbeschleunigern Verwendung.

Die folgenden Beispiele dienen der Erläuterung der Erfindung. Die Überlegenheit der erfindungsgemäßen Katalysatoren gegenüber den herkömmlichen Trägerkatalysatoren zur hydrierenden Aminierung von Alkoholen auf der Basis von Aluminiumoxid-Trägern, gemäß DE-A-19 53 263, wird insbesondere in den Beispielen 1 und 2 evident. Mit den erfindungsgemäßen Katalysatoren lassen sich im Vergleich zu den herkömmlichen Katalysatoren bei wesentlich niedrigeren Drucken wesentlich höhere Umsätze und im Falle der Beispiele 1 und 2 wesentlich höhere Selektivitäten bei der Morpholinherstellung erzielen. Der Vergleich zwischen dem erfindungsgemäßen Vollkatalysator A und dem nach dem Imprägnierverfahren hergestellten Zirkoniumdioxid-Trägerkatalysator B, welche ansonsten die gleiche Elementzusammensetzung haben, macht deutlich, daß in den erfindungsgemäßen Katalysatoren, nicht einfach der Aluminiumoxid-Träger gegen einen Zirkoniumdioxid-Träger ausgetauscht wurde, sondern, daß die sauerstoffhaltigen Zirkoniumverbindungen Bestandteil der katalytisch aktiven Masse sind und diese Zirkoniumverbindungen in dieser Form wichtig für die vorteilhaften Eigenschaften des Katalysators sind.

Beispiele

Die folgenden Beispiele 1 und 2 wurden mit einem erfindungsgemäß hergestellten Vollkatalysator (Katalysator A), einem erfindungsgemäßen Trägerkatalysator (Katalysator B) und zum Vergleich mit einem Katalysator gemäß DE-A-1953263 (Katalysator C) ausgeführt.

In sämtlichen Beispielen beziehen sich die Ausbeuteangaben auf das umgesetzte Ausgangsmaterial.

5

Katalysatorherstellung

Katalysator A

Eine Lösung aus Zirkonium-, Kupfer(II)-, Cobalt(II)- und Nickel(II)salzen wurde simultan mit Natriumcarbonatlösung einer Dichte von 1,208 kg/l in eine Fällungsapparatur gepumpt, in der sich frisch gefälltes, in Wasser suspendiertes Zirkoniumoxid befand. Der pH-Wert der Lösung wurde während der Fällung auf 6,0 konstant gehalten und nach Verbrauch der Metallsalzlösung auf 7,5 angehoben. Der Niederschlag wurde gewaschen, bei 120°C zur Gewichtskonstanz getrocknet und bei 400°C bis zur Gewichtskonstanz calciniert. Die erhaltene Katalysatorrohmasse wurde vermahlen, mit 3 Gew.% Graphit vermischt, tablettiert und nochmals bei 520°C 3 h calciniert.

Zusammensetzung :

76 Gew.% Zr, ber. als $ZrO_2$

4 Gew.% Cu, Ber. als CuO

10 Gew.% Co, ber. als CoO

10 Gew.% Ni, ber. als NiO

Katalysator B

Eine reiner, durch Extrudieren hergestellter Zirkoniumdioxidträger wurde mit einer Cobalt-, Kupfer- und Nickelnitrat jedoch keine Zirkoniumverbindung enthaltenden Lösung getränkt und nach der Tränkung bei 120°C getrocknet. Dieses Vorgehen wurde mehrmals wiederholt. Nach der abschließenden Calcinierung bei 520°C betrug die Zusammensetzung :

76 Gew.% Zr, ber. als $ZrO_2$

4 Gew.% Cu, ber. als CuO

10 Gew.% Co, ber. als CoO

10 Gew.% Ni, ber. als NiO

Katalysator C

Ein in DE-B-1953263 genannter Katalysator wurde analog der Verfahrensweise bei der Herstellung von Katalysator B durch wiederholte Tränkung eines extrudierten Aluminiumoxidträgers mit der zur Herstellung von Katalysator B verwendeten Cobalt-, Kupfer- und Nickelnitratlösung hergestellt. Nach der Calcinierung bei 520°C hatte der Katalysator die Zusammensetzung :

76 Gew.% Al, ber. als $Al_2O_3$

4 Gew.% Cu, ber. als CuO

10 Gew.% Co, ber. als CoO

10 Gew.% Ni, ber. als NiO

Beispiel 1

Hydrierende Aminierung von Diethylenglykol

Ein mit 500 $cm^3$ des entsprechenden, vorreduzierten Katalysators gefüllter Reaktor wurde stündlich mit 90 $cm^3$ Diethylenglykol und 350 $cm^3$ flüssigem Ammoniak beschickt. Die Katalysatortemperatur wurde auf eine Temperatur von 200°C und der Druck im Reaktor, durch gleichzeitiges Aufpressen von Wasserstoff, auf 30 bar eingestellt. Aus dem Reaktionsaustrag wurde nach dessen Entspannung überschüssiges Ammoniak abdestilliert. Die Analyse der mit den Katalysatoren A, B und C erhaltenen Reaktionsausträge ergab die in Tabelle 1 aufgeführten Daten.

Tabelle 1

|  | Katalysator A | Katalysator B | Katalysator C |
|---|---|---|---|
| Umsatz von Diethylenglykol [%] | 89,5 | 71,1 | 49,7 |
| Ausbeute an |  |  |  |
| a) Aminoethoxyethanol [%] | 10,0 | 22,7 | 65,9 |
| b) Morpholin [%] | 82,5 | 58,3 | 28,2 |

Beispiel 2

Hydrierende Aminierung von Diethylenglykol

Die Umsetzung wurde analog zu Beispiel 1 ausgeführt, jedoch wurde ein Druck von 200 bar im Reaktionsgefäß eingestellt. Mit den Katalysatoren A, B und C wurden die in Tabelle 2 aufgelisteten Ergebnisse erzielt.

Tabelle 2

|  | Katalysator A | Katalysator B | Katalysator C |
|---|---|---|---|
| Umsatz von Diethylenglykol [%] | 99,7 | 85,8 | 81,5 |
| Ausbeute an |  |  |  |
| a) Aminoethoxyethanol [%] | 0,5 | 15,6 | 21,9 |
| b) Morpholin [%] | 92,8 | 62,9 | 66,7 |

Beispiel 3

Hydrierende Aminierung von n-Butanol

Analog Beispiel 1 wurden am Katalysator A stündlich 90 cm³ n-Butanol mit 300 cm³ flüssigem Ammoniak bei einer Temperatur von 180°C und bei einem Druck von 30 bar umgesetzt.
Umsatz von n-Butanol : 92%.

Ausbeute :

a)     n-Butylamin 51%
b)     Di-n-Butylamin 43%
c)     Tri-n-Butylamin 5%.

Beispiel 4

Hydrierende Aminierung von Tridecanol

Der Versuch von Beispiel 3 wurde wiederholt, wobei anstelle von n-Butanol n-Tridecanol als Alkohol umgesetzt wurde.
Umsatz von Tridecanol : 99%.

Ausbeute :

a)     n-Tridecylamin : 19%
b)     Di-n-Tridecylamin : 72%
c)     Tri-n-Tridecylamin : 8%.

Beispiel 5

Hydrierende Aminierung von Ethanolamin

Analog Beispiel 1 wurden am Katalysator A stündlich 30 cm$^3$ Ethanolamin mit 300 cm$^3$ flüssigem Ammoniak bei einer Temperatur von 220°C und bei einem Druck von 30 bar umgesetzt.
Umsatz von Ethanolamin : 100%.
Ausbeute an Piperazin : 92%.

Beispiel 6

Hydrierende Aminierung von 2-Dimethylaminoethanol 1

In einem Reaktor wurden 1000 cm$^3$ vorreduzierter Katalysator A stündlich mit 100 cm$^3$ 2-Dimethylamino-ethanol-1 und 2000 cm$^3$ flüssigem Ammoniak belastet. Während der Umsetzung wurde eine Temperatur von 120°C und durch Aufpressen von Wasserstoff ein Druck von 250 bar eingestellt. Aus dem Reaktionsaustrag wurde überschüssiges Ammoniak abdestilliert.
Umsatz von 2-Dimethylaminoethanol-1 : 90%.
Ausbeute : 2-Dimethylaminoethylamin-1 : 84%.

Beispiel 7

Hydrierende Aminierung von N-Ethyldiethanolamin

Durch einen Festbettreaktor, der mit 5 l Katalysatorfüllung, bestehend aus 60 Volumenteilen Katalysator A und 40 Volumenteilen Füllkörpern beschickt worden war, wurden in der Rieselfahrweise bei einer Temperatur von 200°C 0,1 l N-Ethyldiethanolamin/l Katalysator und Stunde und 5 l flüssiges Ammoniak/Stunde geleitet. Der Druck im Reaktor wurde durch Aufpressen von Wasserstoff auf 250 bar eingestellt. Nach beendeter Reaktion wurde auf 20 bar entspannt und überschüssiges Ammoniak abdestilliert. Anschließend wurde auf Atmosphärendruck entspannt und das Reaktionsgemisch fraktionierend destilliert. Bei der Destillation wurde N-Ethylpiperazin in 99%iger Reinheit gewonnen. Der Umsatz war vollständig.
Ausbeute : 79%.

Beispiel 8

Hydrierende Aminierung von N-Butyldiethanolamin

N-Butylpiperazin wurde analog Beispiel 7 aus N-Butyldiethanolamin hergestellt und wurde in 99,5%iger Reinheit erhalten. Der Umsatz war vollständig.
Ausbeute : 75%.

## Patentansprüche

### Patentansprüche für folgende Vertragsstaaten : BR, CH, DE, FR, GB, IT, NL, SE, LI

1. Katalysatoren, deren aktive Masse neben 20 bis 85 Gew.% sauerstoffhaltigen Zirkoniumverbindungen, berechnet als $ZrO_2$, 1 bis 30 Gew.% sauerstoffhaltige Verbindungen des Kupfers, berechnet als CuO und jeweils 1 bis 40 Gew.% sauerstoffhaltige Verbindungen des Cobalts und Nickels, berechnet als CoO bzw. NiO, enthält.

2. Katalysatoren nach Anspruch 1, deren aktive Masse neben 70 bis 80 Gew.% sauerstoffhaltigen Zirkoniumverbindungen, berechnet als $ZrO_2$, 1 bis 10 Gew.% sauerstoffhaltige Verbindungen des Kupfers, berechnet als CuO und jeweils 5 bis 20 Gew.% sauerstoffhaltige Verbindungen des Cobalts und Nickels, berechnet als CuO, bzw. NiO, enthält.

3. Katalysatoren nach den Ansprüchen 1 oder 2, deren aktive Masse durch eine gemeinsame Fällung der wasserlöslichen Verbindungen der genannten Elemente mittels Mineralbasen und anschließende Trocknung und Temperung des erhaltenen Niederschlags erhältlich ist.

4. Katalysatoren nach den Ansprüchen 1 oder 2, deren aktive Masse durch die folgenden Maßnahmen

a)     Ausfällen eines Teils der Zirkoniumkomponente aus einer wäßrigen Zirkoniumsalzlösung mittels Mineralbasen ;

b)     gemeinsame Fällung des restlichen Teils der Zirkoniumkomponente mit den übrigen Katalysatorkomponenten aus einer wäßrigen Lösung dieser Komponenten mittels Mineralbasen in Gegenwart des vorher in Stufe a) gefällten Zirkoniumoxidhydrats,

c)     Waschen des Niederschlags, Trocknen und Tempern ;

erhältlich ist.

5. Katalysatoren nach den Ansprüchen 1 oder 2, deren aktive Masse durch eine gemeinsame Fällung von wasserlöslichen Verbindungen des Cobalts, Nickels und Kupfers mittels Mineralbasen in Gegenwart einer vorgelegten, schwerlöslichen sauerstoffhaltigen Zirkoniumverbindung erhältlich ist.

6. Verfahren zur katalytischen, hydrierenden Aminierung von Alkoholen oder Carbonylverbindungen mit Aminierungsmitteln in Gegenwart von Wasserstoff, bei erhöhter Temperatur und bei erhöhtem Druck, dadurch gekennzeichnet, daß man als Katalysator einen Katalysator gemäß den Ansprüchen 1 bis 5 verwendet.

**Patentansprüche für folgenden Vertragsstaat : ES**

1. Verfahren zur Herstellung von Katalysatoren, dadurch gekennzeichnet, daß man die Komponenten der katalytisch aktiven Masse des Katalysators aus wäßrigen Salzlösungen, die diese Elemente enthalten mittels Mineralbasen ausfällt und die erhaltenen Niederschläge trocknet und calciniert, wobei man die Menge der Komponenten so wählt, daß die katalytisch aktive Masse 20 bis 85 Gew.-% sauerstoffhaltige Zirkoniumverbindungen, berechnet als $ZrO_2$, 1 bis 30 Gew.-% sauerstoffhaltige Verbindungen des Kupfers, berechnet als CuO und jeweils 1 bis 40 Gew.-% sauerstoffhaltige Verbindungen des Cobalts und Nickels, berechnet CoO bzw. NiO, enthält.

2. Verfahren gemäß Anspruch 1, dadurch gekennzeichnet, daß man die Cobalt-, Nickel- und Kupferkomponente des Katalysators gemeinsam aus einer dieser Elemente enthaltenden, wäßrigen Salzlösung mittels Mineralbasen in Gegenwart einer Aufschlämmung einer schwer löslichen, sauerstoffhaltigen Zirkoniumverbindung ausfällt und die erhaltenen Niederschläge trocknet und calciniert.

3. Verfahren gemäß Anspruch 1, dadurch gekennzeichnet, daß man die Komponenten der katalytisch aktiven Masse in einer gemeinsamen Fällung aus ihrer wäßrigen Lösung mittels Mineralbasen ausfällt und die erhaltenen Niederschläge trocknet und calciniert

4. Verfahren gemäß Anspruch 1, dadurch gekennzeichnet, daß man

a)     einen Teil der Zirkoniumkomponente aus einer wäßrigen Zirkoniumsalzlösung mittels Mineralbasen ausfällt,

b)     den restlichen Teil der Zirkoniumkomponente mit den übrigen Katalysatorkomponenten aus einer wäßrigen Lösung dieser Komponenten gemeinsam mittels Mineralbasen in Gegenwart des vorher in Stufe a) gefällten Zirkoniumoxidhydrats ausfällt und

c)     den erhaltenen Niederschlag wäscht, trocknet und calciniert.

5. Verfahren nach Anspruch 1, dadurch gekennzeichnet, daß man Katalysatoren herstellt, deren aktive Masse neben 70 bis 80 Gew.-% sauerstoffhaltigen Zirkoniumverbindungen, berechnet als $ZrO_2$, 1 bis 10 Gew.-% sauerstoffhaltige Verbindungen des Kupfers, berechnet als CuO und jeweils 5 bis 20 Gew.-% sauerstoffhaltige Verbindungen des Cobalts und Nickels, berechnet als CuO bzw. NiO, enthält.

6. Verfahren zur katalytischen, hydrierenden Aminierung von Alkoholen oder Carbonylverbindungen mit Aminierungsmitteln in Gegenwart von Wasserstoff, bei erhöhter Temperatur und bei erhöhtem Druck, dadurch gekennzeichnet, daß man als Katalysator einen Katalysator, der gemäß den Ansprüchen 1 bis 5 hergestellt wurde, verwendet.

**Claims**

**Claims for the following Contracting States : BE, CH, DE, FR, GB, IT, NL, SE, LI**

1. A catalyst whose active material contains, in addition to from 20 to 85% by weight, calculated as $ZrO_2$, of oxygen containing zirconium compounds, from 1 to 30% by weight, calculated as CuO, of oxygen-containing compounds of copper and from 1 to 40% by weight each, calculated as CoO or NiO, respectively, of oxygen-containing compounds of cobalt and of nickel.

2. A catalyst as claimed in claim 1, whose active material contains, in addition to from 70 to 80% by weight, calculated as $ZrO_2$, of oxygen-containing zirconium compounds, from 1 to 10% by weight, calculated as CuO, of oxygen-containing compounds of copper and from 5 to 20% by weight each, calculated as CuO or NiO, respectively, of oxygen-containing compounds of cobalt and of nickel.

3. A catalyst as claimed in claim 1 or 2, whose active material is obtainable by coprecipitation of the water-soluble compounds of the stated elements by means of mineral bases and subsequent drying and heating of the resulting precipitate.

4. A catalyst as claimed in claim 1 or 2, whose active material is obtainable by the following measures :

a)  precipitation of some of the zirconium component from an aqueous zirconium salt solution by means of a mineral base,

b)  coprecipitation of the remaining part of the zirconium component with the remaining catalyst components from an aqueous solution of these components by means of a mineral base in the presence of the zirconium oxide hydrate precipitated beforehand in stage a), and

c)  washing of the precipitate, drying and heating.

5. A catalyst as claimed in claim 1 or 2, whose active material is obtainable by coprecipitation of water-soluble compounds of cobalt, of nickel and of copper by means of a mineral base in the presence of an initially taken, sparingly soluble oxygen-containing zirconium compound.

6. A process for the catalytic amination of an alcohol or carbonyl compound under hydrogenating conditions with an aminating agent in the presence of hydrogen at elevated temperatures and under superatmospheric pressure, wherein the catalyst used is a catalyst as claimed in claim 1 or 2 or 3 or 4 or 5.

## Claims for the following Contrating States : ES

1. A process for the preparation of a catalyst, wherein the components of the catalytically active material of the catalyst are precipitated from aqueous salt solutions which contain these elements by means of a mineral base, and the resulting precipitate is dried and calcined, the amount of the components being chosen so that the catalytically active material contains from 20 to 85% by weight, calculated as $ZrO_2$, of oxygen-containing zirconium compounds, from 1 to 30% by weight, calculated as CuO, of oxygen-containing compounds of copper and from 1 to 40% by weight each, calculated as CoO or NiO, respectively, of oxygen-containing compounds of cobalt and of nickel.

2. A process as claimed in Claim 1, wherein the cobalt, nickel and copper components of the catalyst are coprecipitated from an aqueous salt solution containing these elements by means of a mineral base in the presence of a suspension of a sparingly soluble, oxygen-containing zirconium compound, and the resulting precipitate is dried and calcined.

3. A process as claimed in claim 1, wherein the components of the catalytically active material are coprecipitated from their aqueous solution by means of a mineral base, and the resulting precipitate is dried and calcined.

4. A process as claimed in claim 1, wherein

a)  some of the zirconium component is precipitated from an aqueous zirconium salt solution by means of a mineral base,

b)  the remaining part of the zirconium component is coprecipitated with the remaining catalyst components from an aqueous solution of these components by means of a mineral base in the presence of the zirconium oxide hydrate precipitated beforehand in stage a), and

c)  the resulting precipitate is washed, dried and calcined.

5. A process as claimed in claim 1, wherein a catalyst is prepared whose active material contains, in addition to from 70 to 80% by weight, calculated as $ZrO_2$, of oxygen-containing zirconium compounds, from 1 to 10% by weight, calculated as CuO, of oxygen-containing compounds of copper and from 5 to 20% by weight each, calculated as CuO or NiO, respectively, of oxygen-containing compounds of cobalt and of nickel.

6. A process for the catalytic amination of an alcohol or carbonyl compound under hydrogenating conditions with an aminating agent in the presence of hydrogen at elevated temperatures and under superatmospheric pressure, wherein the catalyst used is a catalyst which has been prepared as claimed in claim 1 or 2 or 3 or 4 or 5.

**Revendications**

**Revendications pour les Etats contractants suivants : BE, CH, DE, FR, GB, IT, LI, NL, SE**

1. Catalyseurs dont la masse active contient, outre 20 à 85% en poids de composés oxygénés du zirconium, calculés en tant que $ZrO_2$, de 1 à 30% en poids de composés oxygénés du cuivre, calculés en tant que CuO, et de 1 à 40% en poids respectivement de composés oxygénés du cobalt et du nickel, calculés en tant que CoO et NiO.

2. Catalyseurs selon la revendication 1, dont la masse active contient, outre 70 à 80% en poids de composés oxygénés du zirconium, calculés en tant que $ZrO_2$, de 1 à 10% en poids de composés oxygénés du cuivre, calculés en tant que CuO, et de 5 à 20% en poids respectivement de composés oxygénés du cobalt et du nickel, calculés en tant que CoO et NiO.

3. Catalyseurs selon la revendication 1 ou 2, dont la masse active est préparable par une précipitation simultanée des composés hydrosolubles desdits éléments au moyen de bases minérales, suivie de séchage et de traitement thermique du précipité obtenu.

4. Catalyseurs selon la revendication 1 ou 2, dont la masse active est préparable par les dispositions suivantes

    a)    précipitation d'une partie du composant à base de zirconium à partir d'une solution aqueuse de sel de zirconium au moyen de bases minérales ;

    b)    précipitation simultanée de la partie restante du composant à base de zirconium avec les autres composants du catalyseur à partir d'une solution aqueuse de ces composants au moyen de bases minérales, en présence de l'oxyde de zirconium hydraté précédemment précipité dans l'étape a) ;

    c)    lavage du précipité, séchage et traitement thermique.

5. Catalyseurs selon la revendication 1 ou 2, dont la masse active est préparable par une précipitation simultanée de composés hydrosolubles du cobalt, du nickel et du cuivre au moyen de bases minérales, en présence d'un composé oxygéné du zirconium difficilement soluble, introduit au préalable.

6. Procédé d'amination hydrogénante catalytique d'alcools ou de composés carbonylés par des agents d'amination en présence d'hydrogène, à température élevée et sous pression élevée, caractérisé en ce qu'on utilise comme catalyseur un catalyseur selon l'une quelconque des revendications 1 à 5.

**Revendications pour l'Etat contractant suivant : ES**

1. Procédé de préparation de catalyseurs, caractérisé en ce qu'on précipite les composants de la masse catalytiquement active du catalyseur à partir de solutions de sels qui contiennent ces éléments au moyen de bases minérales, puis on sèche les précipités obtenus et on les soumet à un traitement thermique, les quantités des composants étant choisies de telle sorte que la masse catalytiquement active contienne de 20 à 85% en poids de composés oxygénés du zirconium, calculés en tant que $ZrO_2$, de 1 à 30% en poids de composés oxygénés du cuivre, calculés en tant que CuO, et de 1 à 40% en poids respectivement de composés oxygénés du cobalt et du nickel, calculés en tant que CoO et NiO.

2. Procédé selon la revendication 1, caractérisé en ce qu'on précipite simultanément les composants du catalyseur à base de cobalt, de nickel et de cuivre à partir d'une solution aqueuse de sels contenant ces éléments, au moyen de bases minérales et en présence d'une suspension d'un composé oxygéné difficilement soluble du zirconium, puis on sèche les précipités obtenus et on les calcine.

3. Procédé selon la revendication 1, caractérisé en ce qu'on précipite les composants de la masse catalytiquement active en une précipitation simultanée à partir de leur solution aqueuse au moyen de bases minérales, puis on sèche les précipités obtenus et on les calcine.

4. Procédé selon la revendication 1, caractérisé en ce que

    a)    on précipite une partie du composant à base de zirconium à partir d'une solution aqueuse de sel de zirconium au moyen de bases minérales,

    b)    on précipite simultanément la partie restante du composant à base de zirconium avec les autres composants du catalyseur à partir d'une solution aqueuse de ces composants au moyen de bases minérales, en présence de l'oxyde de zirconium hydraté précédemment précipité dans l'étape a) et

    c)    on lave le précipité obtenu, on le sèche et on le calcine.

5. Procédé selon la revendication 1, caractérisé en ce qu'on prépare des catalysateurs dont la masse active contient, outre 70 à 80% en poids de composés oxygénés du zirconium, calculés en tant que $ZrO_2$, de 1 à 10% en poids de composés oxygénés du cuivre, calculés en tant que CuO, et de 5 à 20% en poids respectivement de composés oxygénés du cobalt et du nickel, calculés en tant que CoO et NiO.

6. Procédé d'amination hydrogénante catalytique d'alcools ou de composés carbonylés par des agents d'amination en présence d'hydrogène, à température élevée et sous pression élevée, caractérisé en ce qu'on utilise comme catalyseur un catalyseur selon l'une quelconque des revendications 1 à 5.